(19) Europäisches Patentamt
European Patent Office
Office européen des brevets

(11) Veröffentlichungsnummer: **0 092 097**
**B1**

(12) # EUROPÄISCHE PATENTSCHRIFT

(45) Veröffentlichungstag der Patentschrift: **16.01.85**

(51) Int. Cl.[4]: **C 07 C 47/22,** C 07 C 45/75

(21) Anmeldenummer: **83103308.9**

(22) Anmeldetag: **05.04.83**

(54) **Verfahren zur Herstellung von alpha-Alkylacroleinen.**

(30) Priorität: **14.04.82 DE 3213681**

(43) Veröffentlichungstag der Anmeldung:
**26.10.83 Patentblatt 83/43**

(45) Bekanntmachung des Hinweises auf die Patenterteilung:
**16.01.85 Patentblatt 85/3**

(84) Benannte Vertragsstaaten:
**BE DE FR GB IT NL**

(56) Entgegenhaltungen:
**EP - A - 0 058 927**
**DE - B - 2 855 504**

(73) Patentinhaber: **BASF Aktiengesellschaft, Carl-Bosch-Strasse 38, D-6700 Ludwigshafen (DE)**

(72) Erfinder: **Duembgen, Gerd, Dr., Sudetenstrasse 4, D-6701 Dannstadt-Schauernheim (DE)**
Erfinder: **Fouquet, Gerd, Dr., Maxburgstrasse 2, D-6730 Neustadt (DE)**
Erfinder: **Krabetz, Richard, Dr., Unterer Waldweg 8, D-6719 Kirchheim (DE)**
Erfinder: **Lucas, Ekhart, Dr., Burgunderweg 7, D-6706 Wachenheim (DE)**
Erfinder: **Merger, Franz, Dr., Max-Slevogt-Strasse 25, D-6710 Frankenthal (DE)**
Erfinder: **Nees, Friedbert, Dr., Fichtestrasse 4, D-7513 Stutensee (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 092 097 B1

## Beschreibung

Die Erfindung betrifft ein Verfahren zur Herstellung von α-Alkylacroleinen durch Umsetzung von Alkanalen mit Formaldehyd in Gegenwart von sekundären Aminen und gewünschtenfalls von Säuren in flüssiger Phase unter Überdruck bei einer Temperatur von mehr als 150° C und einer Reaktionszeit von höchstens 25 min.

Es ist bekannt, α-Alkylacroleine durch Mannich-Kondensation von n-Alkanalen mit Formaldehyd mit Hilfe von sekundären Aminen herzustellen

$$R^1-CH_2-CHO + CH_2O \rightarrow CH_2=\underset{\displaystyle R^1}{\underset{|}{C}}-CHO$$

Im allgemeinen werden Temperaturen von über 40° C, Drücke von 1 bis 10 bar und pH-Werke >3 verwendet. Hierbei kondensieren die Alkanale mit Formaldehyd in Gegenwart von sekundären Aminen.

So können Methacrolein bzw. 2-Ethylacrolein aus Propionaldehyd bzw. n-Butyraldehyd und Formaldehyd nach dem in DE-PS Nr. 875194 beschriebenen Verfahren hergestellt werden. In den Beispielen werden Monocarbonsäuren, wie Buttersäure und Stearinsäure, gezeigt. Molverhältnisse von Propionaldehyd / Formaldehyd = 1/1,02 (mol), Propionaldehyd / sek.-Amin = 1/0,038, Propionaldehyd / Säure = 1/0,013, sek.-Amin / Säure = 3/1, Verweilzeiten von 4 bis 6 h und Siedetemperaturen bis 100° C werden gezeigt. Man arbeitet im allgemeinen bei einem pH-Wert >7.

Bei einem ähnlichen Verfahren (DE-OS Nr. 2855504) zur Herstellung von Methacrolein werden Carbonsäuren, nämlich Ameisensäure, Essigsäure und insbesondere Propionsäure, und als sekundäre Amine Dipropylamin, Methylbutylamin, Ethylbutylamin und insbesondere Di-n-butylamin hervorgehoben. Im Beispiel wird eine Ausbeute von 81,7% bei einer Umsetzung bei 30 bis 100° C und 2,5 bar während 120 min erzielt. Temperaturen zwischen 70 und 120° C und Drücke von 2 bis 10 bar, bevorzugt 95 bis 110° C und Drücke zwischen 2 und 4 bar werden angegeben. Als Verweilzeiten werden 30 bis 120 (Beispiel 120) min angegeben. Molverhältnisse Propionaldehyd / Formaldehyd = 1/1 bis 1,5, Propionaldehyd / sek.-Amin = 1/0,02 bis 0,05 (1/0,025) und Propionaldehyd / Säure = 1/0,1 bis 0,02 (1/0,015) werden genannt. Eine entsprechende Umsetzung zeigt auch DE-OS Nr. 3025350 mit ähnlichen Verweilzeiten, Temperaturen und Drücken.

Alle 3 Veröffentlichungen zeigen katalytische Mengen Amine in Verbindung mit Carbonsäuren. Offenbar sind pH-Werte über 7 wesentlich, um bei den geringen Katalysatorkonzentrationen noch ausreichende Reaktionsgeschwindigkeiten zu erhalten bzw. noch höhere Verweilzeiten, die zu verstärkter Dimerisierung des α-Alkylacroleins führen, zu vermeiden. Nachteilig ist, dass unter den genannten Bedingungen erfahrungsgemäss auch beträchtliche Mengen anderer Aldolkondensationsprodukte entstehen, wie auch aus dem Beispiel der DE-OS Nr. 2855504 hervorgeht.

US-PS Nr. 2518416 beschreibt die Kondensation von Alkanalen und Formaldehyd in geschmolzenem Salz RRNH·HX (HX = eine Säure mit der Mindestacidität von Trichloressigsäure), bevorzugt in $(CH_3)_2NH \cdot HCl$, bei 120 bis 300, vorzugsweise 140 bis 220° C, drucklos bzw. mit verringertem Druck. Beispiel 1 zeigt eine Ausbeute von 51% α-Ethylacrolein aus n-Butyraldehyd und Formaldehyd bei 200 bis 220° C und ein Molverhältnis von Salz zu Aldehyd wie 7:1. Die grossen Mengen an Aminsalz bedingen ohne Recycling erhebliche Stoffkosten und Entsorgungsprobleme oder bei Recycling einen hohen Aufbereitungsaufwand.

US-PS Nr. 2639295 beschreibt die Kondensation von Propionaldehyd und Formaldehyd, im Überschussverhältnis 2 bis 6/1, in Gegenwart von bis zu 0,25 Eq RRNH·HX (RRNH = sek.-Amin; HX = insbesondere HCl, HBr, $H_2SO_4$ und $H_3PO_4$) bei im allgemeinen 80 bis 130° C und pH 4 bis 6, wobei der überschüssige Propionaldehyd abdestilliert wird. Die höchste in den Beispielen angegebene Reaktionstemperatur beträgt 115° C. Das Ausbeute-Optimum (Beispiel 8) von 92,5%, bezogen auf Formaldehyd, erzielt man mit einem Verhältnis von Propionaldehyd / Formaldehyd = 5/1 mol und einer 10%igen essigsauren Piperidin·HCl-Lösung. Die Umsetzung wird im allgemeinen in Kolonnen durchgeführt (Beispiele). Wie die Beispiele zeigen, erfolgt die Reaktion im Gegenstrom von Ausgangsstoffen und siedender Katalysatorlösung. Die Raum-Zeit-Ausbeute (Beispiel 8) ist unbefriedigend. Der Überschuss an Propionaldehyd ist wegen des hohen Stoffkreislaufes unökonomisch und die höhere Selektivität bezüglich Formaldehyd wird mit unvermeidlichen Verlusten an Propionaldehyd, u.a. durch Kondensation zu Methylpentenal, erkauft. Die vorgeschlagene Regenerierung des Katalysators durch Erhitzen auf 150-300° C ist bezüglich der gebildeten inaktiven tert.-Amine $RRNCH_3$ unwirksam.

Ein ähnliches, aber 2stufiges Verfahren beschreibt US-PS Nr. 2848499 in der kontinuierlichen Kondensation von Propionaldehyd / Formaldehyd / RRNH·HX im Verhältnis 1/1/2 bis 5 bei 105 bis 120° C unter Druck. Als Säuren werden genannt HCl, $H_2SO_4$, $H_3PO_4$ und Essigsäure. Das einzige Beispiel ist mit HCl beschrieben und zeigt, dass mit einem Verhältnis von Propionaldehyd / Formaldehyd / $(CH_3)_2NH \cdot HCl$ = 1/1,036/2,5 bei 111° C ein Umsatz von 98,1% Propionaldehyd und eine Selektivität von 99,6%, bezogen auf Propionaldehyd, bzw. 99%, bezogen auf Formaldehyd, erzielt wird. Das Beispiel zeigt eine Gesamtverweilzeit in den beiden Stufen von mindestens 45 min. Das Verfahren arbeitet mit einem hohen Überschuss an Aminsalz, der die bei US-PS Nr. 2518416 bereits genannten Nachteile mit sich bringt.

„Russ. Chem. Rev.“, *33* (1964), 314 beschreibt die Umsetzung von Propionaldehyd / Formaldehyd / $(C_2H_5)_2NH \cdot HCl$ = 1/1/1 bei pH 6 bis 7 während 20 min bei 45° C und die anschliessende Freisetzung des Methacroleins.

Diese zweistufige Verfahrensweise soll hohe

Selektivitäten ergeben. Eine starke Abhängigkeit vom pH wurde festgestellt, beim Optimum pH 6 bis 7 könne man im Gegensatz zu pH 3 bis 4, wo man bei 100 bis 120° C umsetzen müsse, bei so tiefer Temperatur arbeiten, dass ohne Polymerisationsverluste hohe Selektivitäten erreicht werden. Es wird ausdrücklich festgestellt, dass vorgängige Verfahren dagegen weit vom Optimum entfernt sind. Streuende, schwierig reproduzierbare bzw. unbefriedigende Ausbeuten ergeben sich auf jene Weise. Die Autoren unterstreichen daher die Bedeutung der Bedingungen.

Alle genannten Verfahren sind bezüglich gleichzeitig optimaler Werte in den Ausbeuten, dem Katalysatoraufwand, den Raum-Zeit-Ausbeuten, dem technisch einfachen Betrieb unbefriedigend; DE-PS Nr. 875194 und DE-OS Nr. 2855504 verlaufen nicht ausreichend selektiv und rasch; US-PS Nrn. 2518416, 2639295, 2848499 sind verfahrenstechnisch sehr kompliziert (Einsatz hoher Überschüsse einzelner Komponenten, erschwerte Bedingungen) bzw. liefern ebenfalls schlechte Ergebnisse; das in „Russ. Chem. Rev." (loc. cit.) beschriebene Verfahren ergibt zwar befriedigende Ausbeuten, ist aber wegen des hohen Katalysatoreinsatzes nachteilig.

Es wurde nun gefunden, dass α-Alkylacroleine der Formel

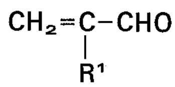 (I)

worin $R^1$ einen aliphatischen Rest bedeutet, durch Umsetzung von Alkanalen der Formel

$$R^1-CH_2-CHO \qquad (II)$$

worin $R^1$ die vorgenannte Bedeutung besitzt, mit Formaldehyd in Gegenwart von sekundären Aminen und gewünschtenfalls von Säure bei Temperaturen zwischen 150 und 300° C vorteilhaft hergestellt werden, wenn die Umsetzung in flüssiger Phase unter Überdruck bei einer Temperatur von mehr als 150° C und einer Reaktionszeit von höchstens 25 min durchgeführt wird.

Die Umsetzung kann für den Fall der Verwendung von Propanal durch die folgende Formel wiedergegeben werden

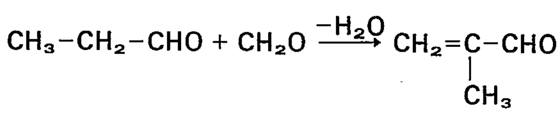


Die Erfindung berücksichtigt, dass nicht nur die Ausbeute, sondern darüber hinaus noch weitere zum Teil sogar wichtigere Ergebnisse der Umsetzung für ein wertvolles Verfahren in Betracht gezogen werden müssen. Im Hinblick auf den Stand der Technik liefert das Verfahren nach der Erfindung eine Kombination vergleichsweise optimaler Ergebnisse, z.B. hohe Selektivität bezüglich beider Komponenten (Alkanal, Formaldehyd) bei fast quantitativem Umsatz, hohe Raum-Zeit-Ausbeute, hohe Katalysatorleistung Q=Mole α-Alkylacrolein / sek.-Amin-Äquivalent, gute Produktqualität (direkte Weiterverarbeitbarkeit), geringer technischer Aufwand, Ausschluss gravierender Toxizitätsprobleme. Alle diese Ergebnisse sind im Hinblick auf die bekannten Verfahren überraschend.

Das Verfahren hebt sich von den bekannten Methoden besonders überraschend durch eine extreme Steigerung der Raum-Zeit-Ausbeuten bei hervorragender Selektivität ab. Es ermöglicht eine technisch und wirtschaftlich vorteilhafte Produktion von α-Alkylacroleinen hoher Reinheit und ist daher u.a. vorzüglich brauchbar zur Produktion von Methacrolein, das bereits als Rohdestillat (mit einem Gehalt von 2 bis 3% Wasser) zur Herstellung von Methacrylsäure durch Oxidation mit $O_2$ sehr gut geeignet ist.

Es ist überraschend, dass das erfindungsgemässe Verfahren bei Verwendung der niedrigen Mengen an Amin und wesentlich kürzeren Reaktionszeiten hohe Umsätze erlaubt und trotz hoher Temperaturen α-Alkylacroleine in hohen Ausbeuten und nahezu nebenproduktfrei in einfachen Reaktoren, z.B. Reaktionsrohren, liefert, ohne dass ein grosser Überschuss an Formaldehyd oder dem entsprechenden Alkanal eingesetzt werden muss.

Auch war es im Hinblick auf „Russ. Chem. Rev." (loc. cit.) nicht zu erwarten, dass bei Temperaturen oberhalb 150° C nahezu keine Nebenprodukte entstehen. Nach „Kinet. Catal.", Bd. 4 (1963), S. 464 treten Polymerisation und Nebenproduktbildung selbst unter Stickstoff und unter Verwendung eines Inhibitors ein. Auch sind die Verfahren nach DE-OS Nrn. 2855504 und 3025350 auf Temperaturen von 70 bis 120° C beschränkt, wobei aber als obere Grenze des bevorzugten Temperaturintervalls die tiefere Temperatur von 110° C genannt wird. US-PS Nr. 2518416 zeigt bei den hohen Temperaturen von 200 bis 220° C (Beispiel 1) eine Ausbeute für die Herstellung von α-Ethylacrolein von lediglich 51%.

Die Alkanale II können mit Formaldehyd in stöchiometrischen Mengen, im Unterschuss oder auch im Überschuss, in der Regel in einer Menge von 0,9 bis 1,5, vorzugsweise 0,95 bis 1,2, vorteilhaft 1 bis 1,1, insbesondere 1 mol Ausgangsstoff II je Mol Formaldehyd umgesetzt werden. Das Formaldehyd wird zweckmässig in wässeriger, vorteilhaft in 20- bis 60gew.-%iger Lösung verwendet.

Bevorzugte Ausgangsstoffe II und dementsprechend bevorzugte Endstoffe I sind solche, in deren Formeln $R^1$ einen n-Alkylrest mit 1 bis 8, vorzugsweise 1 bis 4 Kohlenstoffatomen bedeutet. Die vorgenannten Reste können noch durch unter den Reaktionsbedingungen inerte Gruppen, z.B. Alkoxygruppen oder Alkylgruppen mit 1 bis 4 Kohlenstoffatomen, substitutiert sein.

So kommen als Ausgangsstoffe II beispielsweise in Frage: Propanal, n-Butanal, 3-Methylbutanal, n-Pentanal, n-Hexanal, 3-Methylhexanal, 3-Ethylpentanal, 4-Methylhexanal, n-Heptanal, n-Octanal, n-Nonanal.

Die Umsetzung kann in Abwesenheit oder vorteilhaft in Gegenwart von Säure, in der Regel anorganischer Säure, organischer Mono-, Di- bzw. Polycarbonsäure, bevorzugt Monocarbonsäure,

insbesondere aliphatischer Monocarbonsäure, durchgeführt werden.

Als Carbonsäuren verwendet man zweckmässig aliphatische Monocarbonsäure mit 1 bis 10, vorzugsweise 2 bis 4, oder Di- und Polycarbonsäuren mit 2 bis 10, vorzugsweise 2 und 4 bis 6 Kohlenstoffatomen. Die Dicarbonsäuren und Polycarbonsäuren können aromatische, araliphatische und bevorzugt aliphatische Carbonsäuren sein. Geeignet sind z.B. Essig-, Propion-, Methoxyessig-, n-Butter-, Isobutter-, Oxal-, Bernstein-, Wein-, Glutar-, Adipin-, Malein-, Furmarsäure. Andere organische Säuren sind prinzipiell ebenfalls verwendbar, in der Regel aber aus Preisgründen weniger zweckmässig.

Als anorganische Säuren werden in der Regel Schwefel- und Phosphorsäure eingesetzt. Es können auch Säuregemische verwendet werden. Je Mol Alkanal II verwendet man zwischen 0 und 0,25, vorteilhaft von 0,01 bis 0,1, bevorzugt 0,02 bis 0,05 Eq Säure.

Als Amine kommen zweckmässig solche der Formel

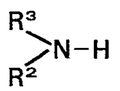 (III)

in Betracht, bei denen $R^2$ und $R^3$ gleich oder verschieden sind und jeweils einen Alkylrest mit 1 bis 10, vorteilhaft 1 bis 8, insbesondere 1 bis 4 Kohlenstoffatomen, die noch durch Ether-, Hydroxy-, sekundäre, tertiäre Aminogruppen, insbesondere durch 1 bis 2 dieser Gruppen, substituiert sein können, einen Aralkylrest mit 7 bis 12 Kohlenstoffatomen, einen Cycloalkylrest mit 5 bis 7 Kohlenstoffatomen bedeuten, $R^2$ und $R^3$ auch mit dem benachbarten Stickstoff Glieder eines heterocyclischen, vorteilhaft 5 bis 7gliedrigen Ringes, der noch ein weiteres Stickstoffatom und/oder ein Sauerstoffatom enthalten und durch Hydroxyalkyl- oder Alkylgruppen mit 1 bis 4 Kohlenstoffatomen substituiert sein kann, bezeichnen können.

Als Amine III kommen beispielsweise in Betracht: Dimethylamin, Diethylamin, Methylethylamin, Methylpropylamin, Dipropylamin, Dibutylamin, Diisopropylamin, Diisobutylamin, Methylisopropylamin, Methylisobutylamin, Methylsek.-butylamin, Methyl-(2-methylpentyl)amin, Methyl-(2-ethylhexyl)amin, Pyrrolidin, Piperidin, Morpholin, N-Methylpiperazin, N-Hydroxyethylpiperazin, Piperazin, Hexamethylenimin, Diethanolamin, Methylethanolamin, Methylcyclohexylamin, Methylcyclopentalamin, Dicyclohexylamin; entsprechende Gemische.

Je Mol Alkanal II verwendet man 0,001 bis 0,25 Eq Amin, vorteilhaft 0,01 bis 0,1, bevorzugt 0,02 bis 0,05.

Das Verhältnis der Äquivalente Amin zu Säure wird vorzugsweise so gewählt, dass ein pH-Wert von 2,5 bis 7 resultiert. Die Umsetzung wird stets bei einer Temperatur von mehr als 150, vorteilhaft zwischen 150 und 300, bevorzugt von 160 bis 220, insbesondere 160 bis 210°C, stets unter Überdruck, diskontinuierlich oder vorteilhaft kontinuierlich durchgeführt. Der Reaktionsdruck beträgt mehr als 1 bar, in der Regel zwischen 1 und 300, zweckmässig von 5 bis 300, vorteilhaft von 10 bis 150, bevorzugt 20 bis 100, insbesondere 40 bis 80 bar. Druck und Temperatur werden so eingestellt, dass die Umsetzung stets unterhalb des Siedepunktes des Reaktionsgemisches erfolgt.

Die Verweilzeit bzw. Reaktionszeit beträgt höchstens 25, zweckmässig 0,01 bis 25, vorteilhaft 0,015 bis 10, bevorzugt 0,03 bis 1, insbesondere 0,05 bis 0,5, vorzugsweise 0,05 bis 0,3 min. Als Reaktor wird bei Verweilzeiten unter 10 min vorteilhaft ein Rohrreaktor eingesetzt.

In den Reaktionsgemischen können neben Wasser zusätzlich organische Lösungsmittel wie z.B. Propanol, Dioxan, Tetrahydrofuran, Methoxyethanol enthalten sein.

Die Reaktion kann wie folgt durchgeführt werden: Ein Gemisch von Ausgangsstoff II, Amin III, Formaldehyd und zweckmässig Wasser und/oder Säure wird während der Reaktionszeit bei der Reaktionstemperatur und dem Reaktionsdruck gehalten.

In einer bevorzugten Ausführungsform wird ein Gemisch (zweckmässig äquimolares Gemisch) aus Formaldehyd und Ausgangsstoff II über einen Wärmetauscher auf die gewünschte Reaktionstemperatur erhitzt und einem Rohrreaktor zugeleitet. In dieses Gemisch wird eine Katalysatorlösung (Lösung des sekundären Amins und einer Säure, zweckmässig in $H_2O$) am Reaktoreingang eingedüst, die gegebenenfalls über einen Wärmetauscher ebenfalls auf die Reaktionstemperatur erwärmt wird. Die stark exotherme Reaktion setzt ein und das Reaktionsgemisch erwärmt sich weiter. Der Druck, unter dem die Reaktion abläuft, wird durch ein Druckhalteventil am Reaktorausgang auf solchen Werten gehalten, dass das Reaktionsgemisch auch bei hohen Temperaturen im Reaktor während der Reaktionszeit noch flüssig bleibt. Nach der Umsetzung wird das Reaktionsgemisch auf Normaldruck entspannt und aufgearbeitet. Bei der Herstellung von Methacrolein aus Propanal und Formaldehyd wird bevorzugt das Reaktionsgemisch einer Kolonne zugeleitet und dort mit Wasserdampf gestrippt. Das Methacrolein verlässt zusammen mit Wasser die Kolonne am Kopf. Das Gemisch wird kondensiert und über ein Phasentrenngefäss in eine obere und eine untere Phase getrennt. Die obere Phase enthält das Methacrolein und wird in einem Behälter gesammelt. Die untere Phase besteht hauptsächlich aus Wasser. Sie wird zur Entfernung des noch darin gelösten Methacroleins wieder in die Kolonne zurückgeführt. Die wässerige Katalysatorlösung wird am Sumpf der Kolonne zusammen mit dem bei der Reaktion gebildeten Wasser und dem Wasser der Formaldehydlösung abgezogen. Für die Weiterverarbeitung kann, wenn sehr wenig Amin eingesetzt wird und deshalb die Rückführung des Katalysators nicht mehr lohnt, die Sumpfflüssigkeit verworfen werden. Bei grösseren Aminkonzentrationen im Sumpfablauf kann aber auch Wasser teilweise destillativ abgetrennt und die Katalysatorlösung wieder in den Reaktor zurückgeführt werden. Ausserdem ist es möglich, den Sumpfablauf so in zwei Teilströme aufzuteilen, dass ein Teilstrom gerade die Menge an Wasser mit sich führt, die bei der Reaktion gebildet und mit den Ausgangsstoffen eingegeben wurde. Dieser Teilstrom wird dann ausgeschleust und der restliche Anteil in den Reaktor zurückgeführt. Wässeriger Formaldehyd und Alkanal II können auch getrennt vorgeheizt und dem Reaktor zugefahren werden. Sofern mit wässerigem Formaldehyd nicht mischbare Alkanale II umgesetzt werden, ist die getrennte Zufuhr zweckmässig.

Die Auftrennung des Reaktionsgemisches durch Destillation oder Wasserdampfdestillation in einer Kolonne stellt nur eines der möglichen Aufarbeitungsverfahren dar. In Frage käme z.B. auch eine Extraktion des Endstoffs I aus der Katalysatorlösung mit einem geeigneten Lösungsmittel, z.B. 2-Ethylhexanol. Bei geringer Löslichkeit des Endstoffs I in der Katalysatorlösung kann auch eine einfache Phasentrennung ausreichend sein.

Die nach dem Verfahren herstellbaren α-Alkylacroleine sind wertvolle Ausgangsstoffe für Farbstoffe, Pharmazeutika und Schädlingsbekämpfungsmittel. Durch Oxidation von Methacrolein gelangt man zu den entsprechenden Methacrylsäuren und Methacrylsäureestern, die bei der Herstellung von Kunststoffen, Acrylgläsern, Formmassen, Profilen, Lacken, Schmierölen, Klebern, Textilhilfsmitteln wertvoll sind. Bezüglich der Verwendung wird auf vorgenannte Literatur und „Ullmanns Encyklopädie der technischen Chemie", (4. Ausgabe), Bd. 16, S. 609 bis 614, verwiesen.

*Beispiel 1*

Als Reaktor wurde ein Reaktionsrohr (4 ml) mit einem Druckhalteventil am Rohrende verwendet. Der Reaktorausgang war mit einer Kolonne verbunden. Das Druckhalteventil war so eingestellt, dass die Reaktion in flüssiger Phase bei einem Druck von 48 bar stattfand.

Aus einem Behälter wurden pro Stunde 1574 ml eines Gemisches aus Propionaldehyd und wässerigem Formaldehyd (37 Gew.-%) mit 661 g Propionaldehyd und 342 g Formaldehyd (Molverhältnis 1:1) in einen Wärmetauscher gepumpt, dort auf 161° C erwärmt und dem Rohrreaktor zugeführt. In diese Mischung wurden aus einem zweiten Behälter entnommene 524 ml/h einer mit einem Wärmetauscher ebenfalls auf 161° C erwärmten wässerigen Katalysatorlösung am Reaktoreingang eingedüst. Die Katalysatorlösung enthielt 3,66 Gew.-% Dimethylamin und 5,27 Gew.-% Essigsäure. Diese Mengen entsprachen, bezogen auf 1 mol eingesetzten Propionaldehyd, Verhältnissen von 0,037 mol Amin bzw. 0,04 mol Essigsäure. Durch die stark exotherme Reaktion stieg die Temperatur an. Während der Umsetzung wurden Reaktionstemperaturen von 161 bis 184° C gemessen. Die Verweilzeit betrug 6,9 s, das pH 5,6. Das Reaktionsgemisch wurde nun entspannt und in der Kolonne mit Wasserdampf gestrippt. Am Kolonnenkopf wurden 809,8 g/h organische Phase mit 96,7 Gew.-% Methacrolein erhalten. Dies entsprach einer Ausbeute von 98,1% der Theorie (bezogen auf den eingesetzten Propionaldehyd) und einer Reaktor-Raum-Zeit-Ausbeute von 195,8 g/l·h. Die organische Phase enthält neben Wasser und Methacrolein als organische Verunreinigungen im wesentlichen 0,46 Gew.-% unumgesetzten Propionaldehyd und 0,29 Gew.-% dimeres Methacrolein. Der Endstoff konnte ohne vorherige Reinigung weiterverarbeitet werden. In einer weiteren Vorlage wurden 1316 g/h des Sumpfablaufes der Kolonne gesammelt.

*Beispiel 2*

Analog Beispiel 1 wurde die Umsetzung mit den folgenden Änderungen durchgeführt:

Druck 50 bar; 600,5 g/h (8,34 mol/h) n-Butyraldehyd, 699,7 g/h Formaldehydlösung (8,63 mol Formaldehyd/h); 132 ml/h Katalysatorlösung (26,3 Gew.-% Dimethylamin und 38 Gew.-% Essigsäure); Molverhältnis Formaldehyd/ n-Butyraldehyd / Dimethylamin / Essigsäure von 1,035:1:0,1:0,1; Verweilzeit 10 s. n-Butyraldehyd und Formaldehyd wurden jeweils über einen Wärmetauscher getrennt auf 170° C erhitzt. Der Katalysatorstrom von 20° C ging als Zulauf zum Reaktoreingang, wo die 3 Ströme zusammentrafen. Während der Umsetzung wurden Temperaturen von 170 bis 193° C gemessen. Man erhielt 703 g/h organische Phase mit 95 Gew.-% 2-Ethylacrolein (95,3% der Theorie, bezogen auf eingesetzten n-Butyraldehyd) und einer Raum-Zeit-Ausbeute von 167 kg/l·h. Die organische Phase enthielt neben Wasser und 2-Ethylacrolein als wesentliche organische Verunreinigung 2,3 Gew.-% Butyraldehyd und 0,4 Gew.-% 2-Ethylhexenal. Sumpfablauf 733 g/h.

*Beispiel 3*

Analog Beispiel 1 wurde die Umsetzung mit den folgenden Änderungen durchgeführt:

2888 g/h äquimolare Mischung (jeweils 20,59 mol) aus Propionaldehyd und Formaldehydlösung; 333 g/h wässerige Katalysatorlösung (10 Gew.-% Dimethylamin und 15,3 Gew.-% Essigsäure); Druck 80 bar; Molverhältnis Formaldehyd / Propionaldehyd / Dimethylamin / Essigsäure von 1:1:0,036:0,041; Vorheiztemperatur (Austauscher) 162° C; während der Umsetzung wurden Reaktionstemperaturen von 189 bis 205° C gemessen; Verweilzeit 6 s; 1452 g/h organische Phase (96,5 Gew.-% Methacrolein); Ausbeute 97,2% der Theorie. Die organische Phase enthielt als organische Verunreinigung unter 0,6 Gew.-% Propionaldehyd und unter 1 Gew.-% dimeres Methacrolein und konnte direkt weiterverarbeitet werden. Sumpfablauf 1769 g/h.

*Beispiele 4 bis 6*

Analog Beispiel 1 wurde die Umsetzung mit den folgenden Änderungen durchgeführt:

Verweilzeit 7 s; 1585 g/h einer auf 160° C erwärmten, äquimolaren (jeweils 11,3 mol/h Aldehyd) Mischung Propionaldehyd / Formaldehydlösung, 520 ml/h auf 160° C vorerwärmte wässerige Katalysatorlösung mit 0,037 mol (bezogen auf 1 mol Propionaldehyd) einer der nachfolgend genannten sekundären Amine in Kombination mit Essigsäure; Druck 50 bar; die weiteren Werte wurden in der folgenden Tabelle zusammengestellt.

*Tabelle*

| Beispiel Nr. | Amin | Mol Amin | Mol Essigsäure | Temperaturen während der Reaktion (°C) | Ausbeute (% der Theorie) |
|---|---|---|---|---|---|
| | | bez. auf 1 mol Propionaldehyd | | | |
| 4 | Diethylamin | 0,037 | 0,042 | 161-186 | 92,3 |
| 5 | Piperazin | 0,037 | 0,063 | 161-188 | 95,8 |
| 6 | Di-n-butylamin | 0,037 | 0,052 | 161-186 | 92,7 |

*Beispiel 7*

Analog Beispiel 1 wurde die Umsetzung mit den folgenden Änderungen durchgeführt:

Reaktorvolumen 5,5 ml; pro Stunde 3480 ml eines Gemisches aus 24,76 mol Propionaldehyd und 24,76 mol Formaldehyd (37%ig, wässerig) und 488 ml wässerige Katalysatorlösung (15 Gew.-% Dimethylamin und 16 Gew.-% Schwefelsäure); Druck 80 bar; Molverhältnis Formaldehyd / Propionaldehyd / Dimethylamin / Schwefelsäure von 1:1:0,072:0,035. Vorheiztemperatur der Aldehydmischung 163° C, der Katalysatorlösung 185° C; während der Umsetzung wurden Reaktionstemperaturen von 182 bis 194° C gemessen; 1729 g/h organische Phase (95,2 Gew.-% Methacrolein); Ausbeute 95% der Theorie; Raum-Zeit-Ausbeute 299 kg/l·h; Verweilzeit 5 s.

*Beispiel 8*

Analog Beispiel 1 wurde die Umsetzung mit den folgenden Änderungen durchgeführt:

Reaktorvolumen 5,5 ml; pro Stunde 3450 ml eines Gemisches aus 24,55 mol Propionaldehyd und 24,55 mol Formaldehyd (37%ig, wässerig) sowie 320 ml einer wässerigen Katalysatorlösung (15 Gew.-% Dimethylamin und 15,2 Gew.-% Oxalsäure); Druck 80 bar; Molverhältnis Formaldehyd / Propionaldehyd / Dimethylamin / Oxalsäure von 1:1:0,046:0,023. Vorheiztemperatur der Aldehydmischung 159° C, der Katalysatorlösung 174° C; während der Umsetzung wurden Reaktionstemperaturen von 180 bis 191° C gemessen; 1708 g/h organische Phase (96,4 Gew.-% Methacrolein); Ausbeute 95,8% der Theorie; Raum-Zeit-Ausbeute 294 kg/l·h; Verweilzeit 5,3 s.

*Beispiel 9*

Analog Beispiel 1 wurde die Umsetzung mit den folgenden Änderungen durchgeführt:

Pro Stunde 1531 ml eines Gemisches aus 10,97 mol Propionaldehyd und 10,97 mol Formaldehyd (37%ig, wässerig) sowie 511 ml einer wässerigen Katalysatorlösung (3,79 Gew.-% Dimethylamin und 6,20 Gew.-% Weinsäure); Druck 50 bar; Molverhältnis Formaldehyd / Propionaldehyd / Dimethylamin / Weinsäure von 1:1:0,039:0,019; Vorheiztemperatur der Aldehydmischung und der Katalysatorlösung 168° C; während der Umsetzung wurden Reaktionstemperaturen von 177 bis 197° C gemessen; 763 g/h organische Phase (95 Gew.-% Methacrolein); Ausbeute 94,4% der Theorie; Raum-Zeit-Ausbeute 181 kg/l·h; Verweilzeit 7 s.

## Patentanspruch

Verfahren zur Herstellung von α-Alkylacroleinen der Formel

$$CH_2=\underset{\overset{|}{R^1}}{C}-CHO \qquad (I)$$

worin $R^1$ einen aliphatischen Rest bedeutet, durch Umsetzung von Alkanalen der Formel

$$R^1-CH_2-CHO \qquad (II)$$

worin $R^1$ die vorgenannte Bedeutung besitzt, mit Formaldehyd in Gegenwart von sekundären Aminen und gewünschtenfalls von Säure bei Temperaturen zwischen 150 und 300° C, dadurch gekennzeichnet, dass die Umsetzung in flüssiger Phase unter Überdruck bei einer Temperatur von mehr als 150° C und einer Reaktionszeit von höchstens 25 min durchgeführt wird.

## Claim

A process for the preparation of an α-alkylacrolein of the formula

$$CH_2=\underset{\overset{|}{R^1}}{C}-CHO \qquad (I)$$

where $R^1$ is an aliphatic radical, by reacting an alkanal of the formula

$$R^1-CH_2-CHO \qquad (II)$$

where $R^1$ has the above meaning, with formaldehyde in the presence of a secondary amine and, if desired, of an acid, at from 150 to 300° C, wherein the reaction is carried out in the liquid phase under superatmospheric pressure at above 150° C for not more than 25 min.

## Revendication

Procédé de préparation d'α-alkylacroléines de la formule

$$CH_2=\underset{\overset{|}{R^1}}{C}-CHO \qquad (I)$$

dans laquelle $R^1$ désigne un groupe aliphatique, par réaction d'alcanals de la formule

$$R^1-CH_2-CHO \quad (II)$$

dans laquelle $R^1$ possède la signification définie, à des températures comprises entre 150 et 300° C, en présence d'amines secondaires et éventuellement d'acides, avec l'aldéhyde formique, caractérisé en ce que la réaction est effectuée en phase liquide, à une température supérieure à 150° C et sous pression accrue, la durée de la réaction ne dépassant pas 25 min.